# EUROPÄISCHE PATENTSCHRIFT

(11) **EP 1 038 024 B1**
(45) Veröffentlichungstag und Bekanntmachung des Hinweises auf die Patenterteilung: **13.06.2007**
(21) Anmeldenummer: 98966274.7
(22) Anmeldetag: 09.12.1998
(51) Int. Cl.: C12Q 1/50

(54) **STABILISIERTES REAGENZ UND VERFAHREN ZUR BESTIMMUNG VON CREATIN-KINASE**
STABILISED REAGENT AND METHOD FOR DETERMINING CREATINE KINASE
REACTIF STABILISE ET PROCEDE DE DETERMINATION DU TAUX DE CREATINE-KINASE

(30) Priorität: 11.12.1997 DE 19755079
(43) Veröffentlichungstag der Anmeldung: 27.09.2000
(73) Patentinhaber: Roche Diagnostics GmbH, 68305 Mannheim (DE)
(72) Erfinder: NAGEL, Rolf, D-68642 Bürstadt (DE); MISTELE, Jürgen, D-68782 Brühl (DE); SCHRÖDER, Norbert, D-68712 Schwetzingen (DE)
(86) Internationale Anmeldenummer: PCT/EP1998/008016
(87) Internationale Veröffentlichungsnummer: WO 1999/029895

(56) Entgegenhaltungen:
- EP-A- 0 426 100
- EP-A- 0 774 514
- EP-A- 0 989 191
- WO-A-95/30769
- DATABASE WPI Section Ch, Week 9912 Derwent Publications Ltd., London, GB; Class B04, AN 99-135572 XP002900428 & JP 10 327895 A (NIPPON SHOJI KK) , 15. Dezember 1998
- PATENT ABSTRACTS OF JAPAN vol. 097, no. 007, 31. Juli 1997 & JP 09 070298 A (SHINOTESUTO:KK), 18. März 1997
- PATENT ABSTRACTS OF JAPAN vol. 011, no. 159 (C-423), 22. Mai 1987 & JP 61 289900 A (UNITIKA LTD;OTHERS: 01), 19. Dezember 1986

## Beschreibung

Die Erfindung betrifft ein verbessertes Verfahren bzw. stabilisiertes Reagenz zur photometrischen Bestimmung der Creatin-Kinase in biologischen Proben, wie insbesondere menschlichem Blutserum oder -Plasma. Das Reagenz ist im wesentlichen dadurch gekennzeichnet, daß es mindestens ein Dithionit und/oder ein Sulfitsalz von Alkali- oder Erdalkaliionen im stöchometrischen Unterschuß enthält.

Die Bestimmung der Creatin-Kinase in Serum oder Plasma spielt eine wichtige Rolle bei der Diagnose des Herzinfarkts. Als Standard-Methode wird dazu ein photometrischer Test verwendet, bei dem in gekoppelten Enzymreaktionen aus Creatinphosphat und Adenosin-5'-diphosphat (ADP) durch die in der Probe enthaltene Creatin-Kinase (CK) neben Creatin Adenosin-5'-triphosphat (ATP) erzeugt wird; mit ATP wird beispielsweise aus Glucose in Gegenwart von Hexokinase (HK) Glucose-6-phosphat gebildet, das in der von der Glucose-6-phosphat-Dehydrogenase (G6P-DH) katalysierten Reaktion unter gleichzeitiger Umwandlung von NAD⁺ oder NADP⁺ in NADH bzw. NADPH zu Gluconat-6-phosphat oxidiert wird:

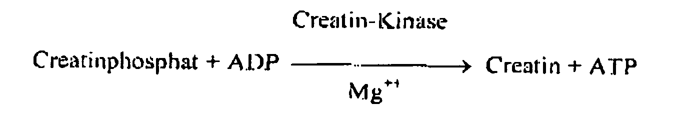

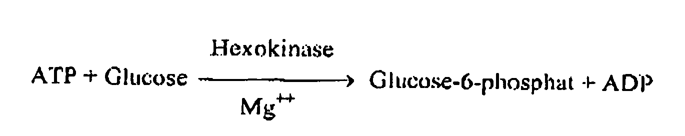

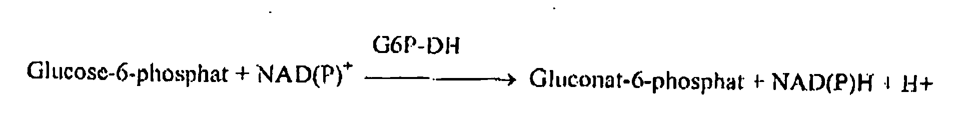

Als Meßgröße dient die in einem vorgegebenen Zeitintervall bei einer bestimmten Temperatur, üblicherweise zwischen 25 und 37°C, durch die Bildung von NAD(P)H erfolgende Extinktionszunahme, die der CK-Aktivität im Probevolumen proportional ist. Damit die CK ihre volle enzymatische Aktivität entfalten kann, wird die Bestimmung üblicherweise in Gegenwart von CK-Aktivatoren, beispielsweise von Thiol-Verhindungen, wie Gluthation, Dithiothreitol, Thioglycerol, 2-Mercaptoethanol und N-Acetyleystein, durchgeführt.

Weiterhin werden dem dem CK-Test zugrundeliegenden Reagenz bevorzugt zusätzlich Inhibitoren für eventuell in der Probe vorhandene Myokinasen (Adenylat-Kinase), wie beispielsweise Adenosin-5'-monophosphat (AMP) und/oder Di-adenosin-pentaphosphat zugesetzt, Trotz dieser Zusätze kann entsprechend folgender Reaktion: 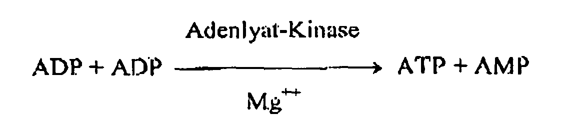 aus ADP unspezifisch ATP entstchen. Dies kann zu einer zusätzlichen, die CK-Bestimmung verfälschenden NAD(P)H-Bildung führen. Die Störung durch unvollständig inhibierte Adenylat-Kinase, die insbesondere bei hämolytischen Proben auftritt, kann dadurch ausgeräumt werden, daß vor dem eigentlichen Start der CK-Reaktion durch die Zugabe von Creatinphosphat die Aktivität der Adenylat-Kinase bestimmt und diese von der Gesamtäktivität (CK und Adenylat-Kinase) substrahiert wird (sogenanntes rate blanking). Wesentlich ist dabei, daß sämtliche für den Nachweis der über Adenylat-Kinase gebildeten ATP-Menge benötigten Komponenten in der ersten Reaktionslösung vorliegen (bis auf Creatinphnsphat).

Für die Bestimmung von Creatin-Kinase-Isoenzymen können dem Reagenz des weiteren noch spezifische Inhibitoren, wie beispielsweise gegen bestimmte CK-Isoenzyme gerichtete Antikörper zugesetzt werden.

Zur Steigerung der Nachweisempfindlichkeit kann das Glucose-6-phosphat bildende und mittels NAD(P)⁺ sowie G6P-DH Glucose-6-phosphat umsetzende Reagenz wahlweise darüber hinaus 6-Phosphogluconolactonase und Gluconat-6-phosphat-Dehydrogenase enthalten; in diesem Fall werden pro Mol gebildetem ATP bzw. Glucose-6-phosphat zwei Mol NADH bzw. NADPH erzeugt (R. Vormbrock und R. Helger, Enzyme 38, Suppl. I (1987), S. 20/21).

Weiterhin ist es beispielsweise möglich, den CK-Nachweis zu führen, indem das aus Creatinphosphat und ADP gebildete ATP mittels Glycerin und Glycerokinase, bevorzugt in Gegenwart von Magnesiumionen, zu Glycerin-3-phosphat umgesetzt wird, welches in Gegenwart von Sauerstoff enzymatisch in Wasserstoffperoxid überführt und in üblicher Weise mittels Peroxidase und Redox-Indikatoren delektiert wird.

Grundsätzlich können sämtliche für die CK-Bestimmung erforderlichen Komponenten, d.h. Enzyme und Substrate, in einem einzigen Reagenz vorliegen, Insbesondere an Analysengeräten wird die Bestimmung jedoch bevorzugt dergestalt durchgeführt, daß man die Probe zunächst mit einem ersten, außer Creatinphosphat sämtliche für die Nachweisreaktion erforderlichen Komponenten und mögliche weitere Hilfsstoffe, wie beispielsweise N-Acetylcystein, Adenylat-Kinase-Inhibitoren sowie gegebenenfalls CK-Isoenzym-Inhibitoren, enthaltenden Teilreagenz für einige Minuten vorinkubiert und anschließend die Nachweisreaktion durch Zugabe eines zweiten, im wesentlichen Creatinphosphat in gepufferter Lösung enthaltenden Teilreagenzes startet.

Neben der Vermeidung einer durch die CK hervorgerufenen NAD(P)H-Bildung in der Phase der in Gegenwart der zugesetzten Thiol-Verbindung ablaufenden Aktivierung des Enzyms ist es auf diese Weise außerdem möglich, etwaige Verfälschungen des Meßergebnisses durch überhöhte, nicht mehr ausreichend durch Adenylat-Kinase-Inhibitoren hemmbare Aktivitäten an Adenylat-Kinase, wie sie beispielsweise in hämolytischen Proben auftreten können, zu verhindern. Hierzu wird nach Vermischen von Probe und dem ersten Teilreagenz eine erste Messung der Bildungsgeschwindigkeit an NAD(P)H durchgeführt und das Ergebnis von der NAD(P)H-Bildungsgeschwindigkeit nach Zugabe des zweiten, Creatinphosphat-haltigen Teilreagenzes abgezogen.

Ein Nachteil für den Anwender solcher Reagenzien besteht nun insbesondere darin, daß die Reagenzien vor Gebrauch erst durch Auflösen von festen Bestandteilen, wie z.B. Lyophili saten, Granulaten oder Tabletten, hergestellt werden müssen und darüber hinaus selbst bei Kühllagerung zwischen etwa 2 his 8 °C nur wenige Tage oder Wochen haltbar sind. Aus Rationalsisierungsgründen im klinischen Labor besteht heute zunehmend der Bedarf an gebrauchsfertigen Reagenzien mit einer Haltbarkeit von mindestens 12 Monaten bei 2 bis 8°C. Dieser Anspruch kann derzeit mangels Stabilität nicht erfüllt werden. Im wesentlichen wird die Instabilität des gebrauchsfertigen Reagenzes durch die Instabilität von N-Acetyleystein (NAC, Aktivator der CK) verursacht.

Das Stabilitätsproblem kann zwar wie in EP 0 686 561 durch Lagerung von NAC (gemeinsam mit NAD(P)) in einer zweiten Lösung bei pH 3.0 gelöst werden, jedoch mit erheblichen Nachteilen. Bei dieser Reagenzformulierung erfolgt die Aktivierung der CK erst mit dem Start der CK-Reaktion. Zu lag-phasenfreien Messung ist daher eine erhöhte Wartezeit vor dem eigentlichen Meßbeginn erforderlich und limitiert dadurch den Meßbereich der Methode erheblich. Als weiterer Nachteil ist die Eliminierung der Adenylat-Kinase-Störung durch rate blanking nicht möglich. In EP 0 774 514 sind zwar Stabilisierungsmaßnahmen für entsprechende Flüssigreagenzien zur Bestimmung von CK beschrieben, wie beispielsweise der Zusatz eines Phosphins und einer Sulfhydrylverbindung, beispielsweise Dithiothreitol, DTT beschrieben. Der Zusatz von DTT ist jedoch mit dem Nachteil verbunden, daß dieser das Hilfsenzym G6P-DH destabilisiert und dadurch der Zusatz einer weiteren Komponente in Form einer Hydroxylamin-Verbindung (wie beispielsweise Carboxymethoxylaminhydrochlorid) zur Stabilisierung der G6P-DH erforderlich macht (EP 0 640 686).

Ferner werden in EP 0 721 986 stabile Reagenzien zur Bestimmung von Creatin-Kinase beschrieben, welche bestimmte SH-Verbindungen wie Thioglycerin (TG), 2-Mercaptoethanol (2ME) oder 2- Mercaptoethansulfonsäure (2MES) in molaren Konzentrationen enthalten. Im Gegensatz zu anderen Aktivatoren hemmen diese in oxidierter Form die CK-Aktivität nicht. Relativ hohe Konzentrationen von entsprechenden SH-Verbindungen können jedoch unerwünschte Nebeneffekte, wie beispielsweise die Inaktivierung von Hilfsenzymen (z.B. G6P-DH), verursachen. Diese Aktivatoren werden demzufolge derzeit auch nicht von entsprechenden Fachkreisen für die Bestimmung der CK-Aktivität empfohlen (z.B. nach 1fcC, DGkCh).

Offen ist zudem die Vergleichbarkeit entsprechender Testsysteme speziell unter dem Aspekt der Isoenzyme und CK verschiedener Spezies.

Der nächstliegende Stand der Technik ist in JP-A-09-070298 und WO 95/30769 beschrieben. JP-A-09-070298 offenbart die Verwendung eines stabilisierten Reagenzes, das eine Oxosäure mit einem zentralen Schwefel-Atom in Kombination mit einer Thiol-haltigen Verbindung als CK-Aktivator beinhaltet, für die Bestimmung der CL-Aktivität. Das Reagenz kann aus zwei Teilreagenzien bestehen, wobei die Oxosäure entweder nur im selben Teilreagenz wie der CK-Aktivator, oder in beiden Teilreagenzien vorhanden ist. Die Verwendung der Oxosäure als Stabilisator lediglich in demjenigen der beiden Teilreagenzien, welches nicht den CK-Aktivator enthält, wird nicht offenbart.
WO 95/30769 beschreibt die Verwendung der organischen Schweißverbindungen Dimethylsulfoxid, Dimethylthiourea, N-2-Mercaptopropionyl Glycine und Glutathion für die Stabilisierung einer Reagenzlösung zur Bestimmung der CK. Die Lösung enthält eine Thiolenthaltende Verbindung, z.B. A-Acetyl Cystein (NAC) als Aktivator. Jedoch wird keine Schwefelverbindung als Stabilisator vorgezogen, sondern Mannitol in Konzentrationen ab 5 mM.

Insbesondere um die Arbeiten im klinischen Labor unter zunehmendem Kostendruck weiter zu rationalisieren, besteht heute mehr und mehr ein Bedarf an stabilisierten Reagenzien, die in gebrauchsfertiger flüssiger Form über mindestens 12 Monate bei 2 bis 8°C, ohne erneute Kalibration, lagerfähig und stabil sind und wobei das Risiko aufgrund bestimmter Zusätze verursachter unspezifischer Reaktionen weitgehend ausgeschaltet ist.

Diese Aufgabe wird erfindungsgemäß gelöst durch ein wie in Anspruch 1 definiertes, Reagenz, welches ein geeignetes Puffersystem, durch CK umsetzbare Substrate bzw. entsprechende Coenzyme, einen CK-Aktivator sowie die für eine oder mehrere enzymatische Folgereaktionen erforderlichen Komponenten und mindestens eine Dithionit und/oder ein Sulfitsalz von Alkali- oder Erdalkaliionen molarem Unterschuß zum CK-Aktivator enthält. Entsprechende CK-Aktivatoren sind dem Fachmann bekannt. Erfindungsgemäß ist ein Reagenz bevorzugt, weiches als durch CK umsetzbare Substrate Creatinphosphat und Adenosin-5'-Diphosphat (ADP) enthält- Besonders bevorzugt ist ein Reagenz, das Glucose, ein Glucose-6-Phosphat bildendes Enzym, wie beispielsweise Hexokinase, ADP, Creatinphosphat, einen CK-Aktivator, gegebenenfalls Adenosin-5'-Monophosphat, ein Coenzym in oxidierter Form, wie NAD⁺, NADP⁺ oder diesbezügliche Derivate, eine G6P-Dehydronase und gegebenenfalls einen oder mehrere Adenylat-Kinase-Inhibitoren und/oder weitere Aktivitoren und mindestens eine Dithionit und/oder ein Sulfitsalz von Alkali- oder Erdalkaliionen im stöchometrischen Unterschuß enthält. Insbesondere haben sich entsprechende Dinatriumsalze zur Stabilisierung von Reagenzien zur CK-Bestimmung als vorteilhaft erwiesen.

Die Konzentration der erfindungsgemäßen Schwefelkomponente im Reagenz liegt in der Größenordnung des zu bestimmenden Enzyms (CK) und beträgt maximal 20 mmol/l. Bevorzugt sind Konzentrationen zwischen ca. 0,01 und 5 mmol/l besonders bevorzugt 0,1 bis 1,0 mmol/l der Schwefelkomponente.

Alle übrigen Komponenten sowie gegebenenfalls weitere Hilfsstoffe, einschließlich der gegebenenfalls zuzusetzenden 6-Phosphogluconolactonase und Gluconat-6-phosphat-Dehydrogenase, werden in für den Fachmann üblichen Konzentrationen verwendet.

Eine besondere Ausführungsform des erfindungsgemäßen Reagenzes ist, wenn die für die CK-Bestimmung erforderlichen Komponenten auf zwei separate Teilreagenzien verteilt vorliegen, wobei im ersten Teilreagenz der CK-Aktivator und sämtliche für die CK-Bestimmung benötigten Substanzen und Enzyme bis auf Creatinphosphat und im zweiten Teilreagenz Creatinphosphat und beispielsweise Glucose enthalten sind. Die erfindungsgemäße Schwefelkomponente liegt dabei im zweiten Teilreagenz nicht jedoch im ersten Teilreagenz vor. Als vorteilhaft hat sich erwiesen, wenn die Schwefelverbindung dem komplexeren ersten Reagenz, welches auch den CK-Aktivator enthält, erst unmittelbar vor der Messung zugefügt wird, z.B. über die zweite Reagenzlösung.

Eine weitere besondere Ausführungsform der Erfindung ist ein Reagenz, welches Glucose bei Kombination von NAD(P)⁺ G6P-DH in einem Teilreagenz in niedriger Konzentration, bevorzugt von ca. 5 mmol/l oder weniger enthält, bevorzugt sind ca. 0,5 bis 1,0 mmol/l Glucose.

Das erfindungsgemäße wäßrige Reagenz zeichnet sich durch besondere Stabilität nach längerer Lagerung zwischen 2 und 8°C, d.h. über einen Zeitraum von mehreren Monaten bis zu 12 Monaten ohne erneute Kalibration und hohe Spezifität aus. Im CK-Funktionstest konnte so lediglich eine geringügige Abweichung gegenüber einem frisch hergestellten Reagenz festgestellt werden, d.h. die CK-Wiederfindungsraten betragen annähernd 100%.

Ein weiterer Gegenstand der Erfindung ist ein Verfahren zu enzymatischen Bestimmung von Creatin-Kinase in biologischem Probenmaterial unter Verwendung des oben näher spezifizierten Reagenzes.

### Legenden zu den Abbildungen:

Abbildung 1: Bestimmung der CK-Aktivität unter Zusatz von Na₂SO₃ (0 bis 4 mmol/l); Lagerzeitraum: 14 Tage, Temperatur: 35°C im Vergleich zu 2 his 8°C.
Abbildung 2: Einfluß von Na₂SO₃ (0 bis 4 mmol/l) auf G6P-DH; Lagerzeitraum: 14 Tage, Temperatur: 35°C im Vergleich zu 2 bis 8°C.
Abbildung 3: Bestimmung der CK-Aktivität unter Zusatz von Na₂SO₃ (1 mmol/l); Lagerzeitraum: 192 Tage, Temperatur: 2 bis 8°C (2 Proben).
Abbildung 4: Einfluß verschiedener Konzentrationen an Na₂SO₃ (0, 1, 2, 3, 4, 5, 10 und 20 mmol/l) auf CK-Aktivität; Temperatur: 2 bis 8°C.
Abbildung 5: Einfluß von Na₂SO₃ (1 mmol/l) im Vergleich zu DTT (10 mmol/l) auf G6P-DH; Lagerzeitraum: 136 Tage, Temperatur: 2 bis 8°C.

Die folgenden Beispiele erläutern die Erfindung weiter:

### Beispiel 1: Bestimmung von CK in Gegenwart von Na₂SO₃

### Reagenzzusatnmensetzung

| | | |
|---|---|---|
| Reagenz 1 (R1): | Imidazol (pH 6,6) | 100 mmol/l |
| | Glucose | 1 mmol/l |
| | Magnesiumacetat | 10 mmol/l |
| | EDTA | 2 mmol/l |
| | ADP | 2 mmol/l |
| | AMP | 5 mmol/l |
| | Di-Adenosin-Pentaphosphat | 10 µmol/l |
| | NADP⁺ | 2 mmol/l |
| | N-Acetylcystein | 20 mmol/l |
| | Hexokinase | 3 U/ml |
| | G6P-Dehydrogenase | 3 U/ml |
| | Na₂SO₃ | 0 bis 4 mmol/l |
| Reagenz 2 (R2): | Capso (pH 9,3) | 20 mmol/l |
| | Glucose | 110 mmol/l |
| | Creatinphosphat | 170 mmol/l |

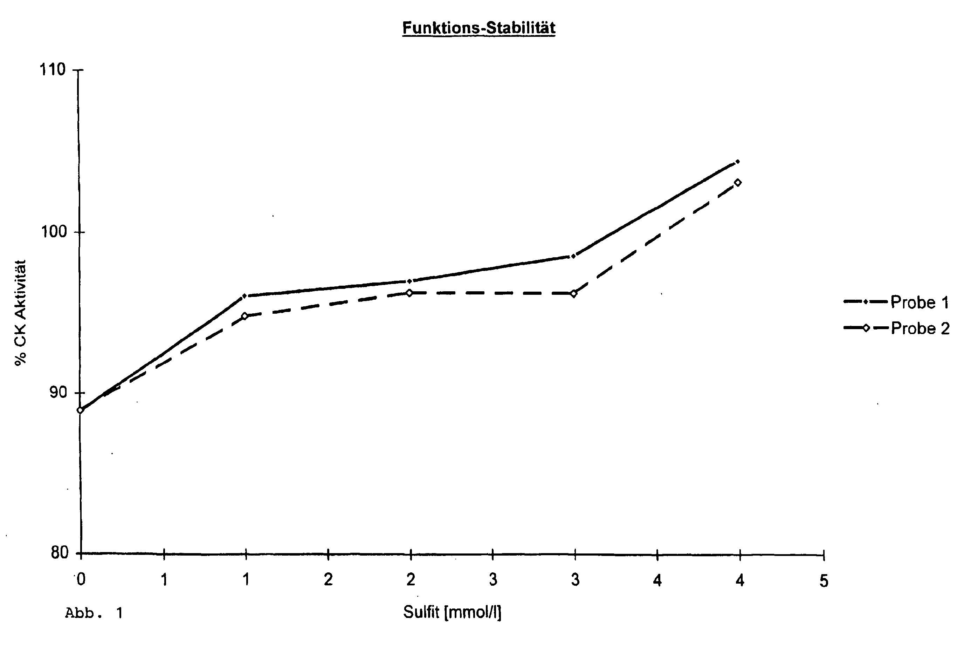

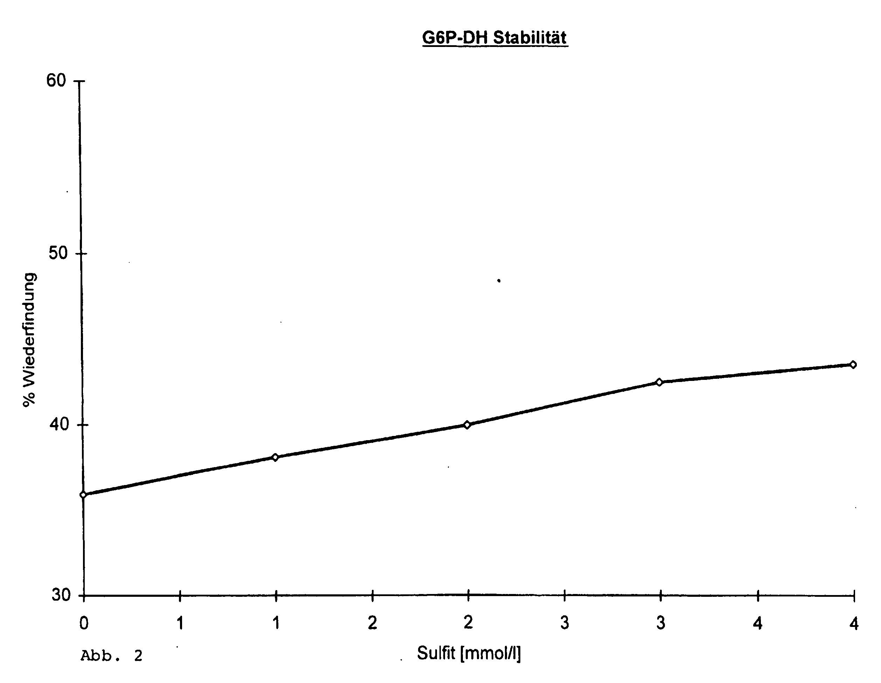

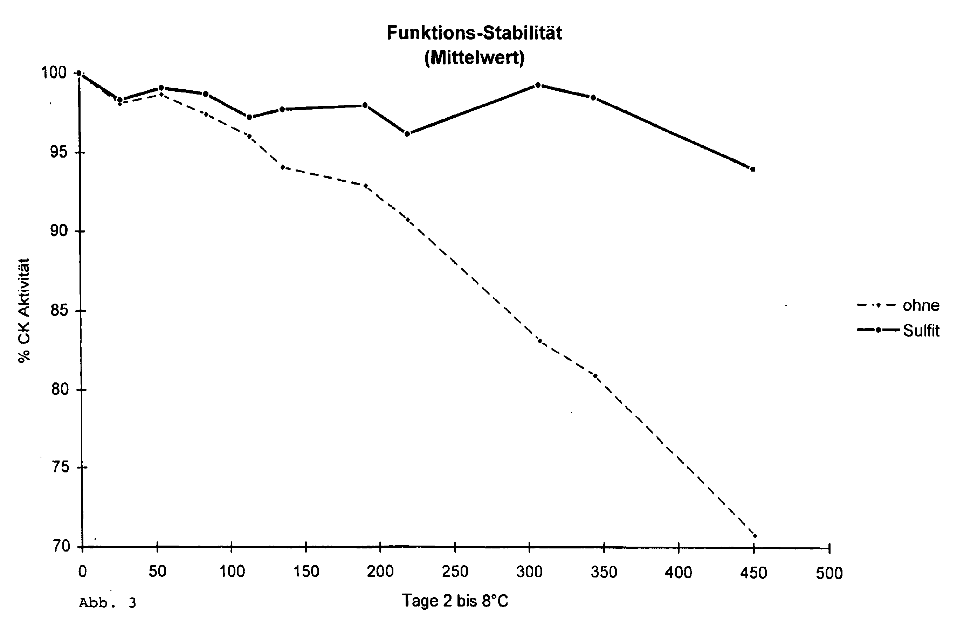

Für die Bestimmung werden 250 µl R1 mit 10 µl Probe vermischt, 5 Minuten inkubiert, mit 50 µl R2 die Reaktion gestartet und nach weiterer Inkubation von 1 Minute die CK-Aktivität gemessen. Die Bestimmung der Enzymaktivität, CK bzw. G6P-DH, erfolgte nach Lagerung bei 2 bis 8°C bzw. 35°C zu unterschiedlichen Zeiten (Tabellen 1-3, Abbildungen 1-3).

**Tabelle 1**

| Sulfit-Mengeneinfluß auf CK-Funktionsstabilität (0 bis 4 mmol/l Na₂SO₃ in Reagenz 1) nach Beispiel 1 | | | | |
|---|---|---|---|---|
| Sulfit | Probe | 14 Tage Lagerung bei | | |
| | | 2 bis 8°C | 35°C | |
| [mmol/l] | | [U/l] | [U/l] | [%] |
| 0 | 1 | 270 | 240 | 88.9 |
| 1 | 1 | 278 | 267 | 96.0 |
| 2 | 1 | 274 | 265 | 97.0 |
| 3 | 1 | 271 | 267 | 98.5 |
| 4 | 1 | 260 | 272 | 104.4 |
| 0 | 2 | 519 | 461 | 88.9 |
| 1 | 2 | 540 | 512 | 94.8 |
| 2 | 2 | 530 | 510 | 96.2 |
| 3 | 2 | 523 | 503 | 96.2 |
| 4 | 2 | 505 | 521 | 103.1 |

**Tabelle 2**

| Sulfit-Mengeneinfluß auf G6P-DH-Stabilität (0 bis 4 mmol/l Na₂SO₃ in Reagenz 1) nach Beispiel 1 | | | |
|---|---|---|---|
| Sulfit | 14 Tage Lagerung bei | | |
| | 2 bis 8°C | 35°C | |
| [mmol/l] | [U/ml] | [U/ml] | [%] |
| 0 | 4.01. | 1.44 | 35.9 |
| 1 | 3.95 | 1.50 | 38.1 |
| 2 | 3.92 | 1.57 | 39.9 |
| 3 | 3.98 | 1.69 | 42.5 |
| 4 | 4.06 | 1.76 | 43.5 |

**Tabelle 3**

| Langzeitstabilitätsfunktionstest CK (0 oder 1 mmol/1 Na₂SO₃ in Reagenz 1) nach Beispiel 1 | | | | | |
|---|---|---|---|---|---|
| Tage | Probe | 0 mmol/l Sulfit | | 1 mmol/l Sulfit | |
| 2 bis 8°C | | [U/l] | [%] | [U/l] | [%] |
| 0 | 1 | 290 | 100,0 | 296 | 100,0 |
| 27 | 1 | 284 | 97,8 | 289 | 97,6 |
| 55 | 1 | 288 | 99,4 | 292 | 98,8 |
| 85 | 1 | 281 | 97,0 | 291 | 98,4 |
| 114 | 1 | 279 | 96,2 | 288 | 97,3 |
| 136 | 1 | 272 | 93,7 | 287 | 97,0 |
| 192 | 1 | 269 | 92,8 | 288 | 97,3 |
| 220 | 1 | 264 | 91,0 | 282 | 95,4 |
| 308 | 1 | 242. | 83,4 | 293 | 99,1 |
| 345 | 1 | 237 | 81,7 | 291 | 98,4 |
| 451 | 1 | 203 | 70,0 | 274 | 92,7 |
| 0 | 2 | 562 | 100,0 | 566 | 100,0 |
| 27 | 2 | 553 | 98,4 | 561 | 99,1 |
| 55 | 2 | 550 | 97,9 | 563 | 99,4 |
| 85 | 2 | 550 | 97,8 | 561 | 99,0 |
| 114 | 2 | 539 | 95,8 | 550 | 97,1 |
| 136 | 2 | 531 | 94,4 | 558 | 98,5 |
| 192 | 2 | 523 | 93,0 | 559 | 98,6 |
| 220 | 2 | 508 | 90,4 | 549 | 96,9 |
| 308 | 2 | 465 | 82,7 | 563 | 99,4 |
| 345 | 2 | 450 | 80,1 | 558 | 98,5 |
| 451 | 2 | 402 | 71,5 | 539 | 95,2 |

### Beispiel 2: Einfluß Menge an Sulfit auf CK-Aktivität

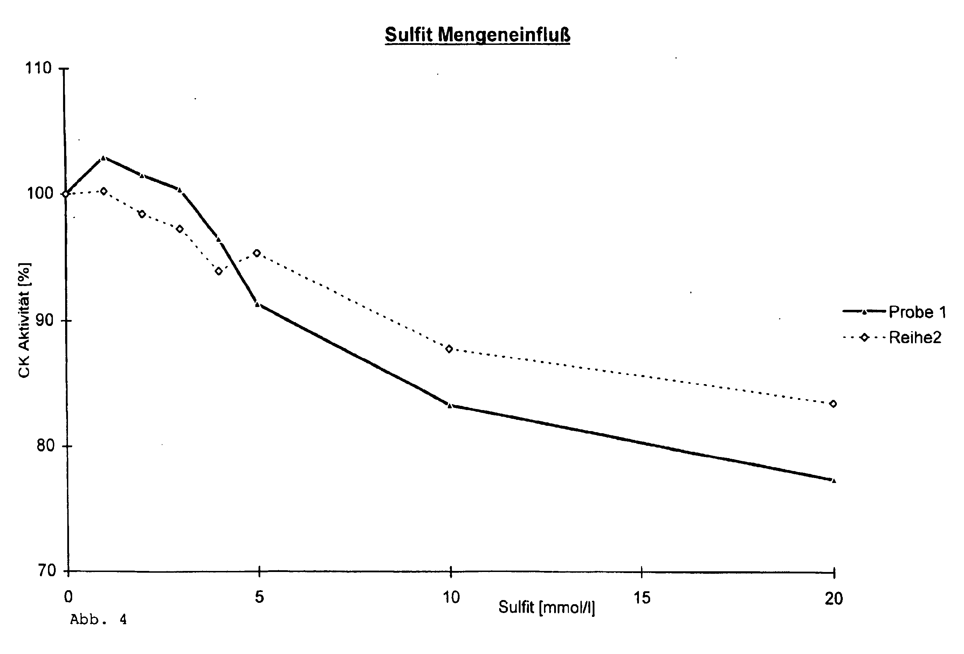

Die Reagenzzusammensetzung und die Testdurchführung erfolgten prinzipiell analog zu Beispiel 1. Variiert wurden lediglich die Reagenz 1 zugesetzte Menge an Natriumsulfit (0 bis 20 mmol/l). Das Ergebnis ist Tabelle 4 bzw. Abbildung 4 zu entnehmen.

**Tabelle 4:**

| Sulfit Mengeneinfluß auf CK-Funktion (0 bis 20 mmol/l Na₂SO₃ in Reagenz 1) | | | | |
|---|---|---|---|---|
| Sulfit | Probe 1 | | Probe 2 | |
| [mmol/l Test] | [U/1] | [%] | [U/1] | [%] |
| 0 | 236 | 100,0 | 529 | 100,0 |
| 1 | 242 | 102,9 | 530 | 100,2 |
| 2 | 239 | 101,4 | 520 | 98,4 |
| 3 | 236 | 100,3 | 514 | 97,2 |
| 4 | 227 | 96,4 | 496 | 93,8 |
| 5 | 215 | 91,3 | 504 | 95,3 |
| 10 | 196 | 83,2 | 464 | 87,7 |
| 20 | 182 | 77,3 | 441 | 83,3 |

### Beispiel 3: Reagenzstabilität in Gegenwart von Dithiothreitol (DTT) (Stand der Technik)

### Reagenzzusammensetzung

| | | |
|---|---|---|
| Reagenz (R): | Imidazol (pH 6,6) | 100 mmol/l |
| | Glucose | 1 mmol/l |
| | Magnesiumacetat | 10 mmol/l |
| | EDTA | 2 mmol/l |
| | Adenosin-5'-Diphosphat | 2 mmol/l |
| | Adenosin-5'-monophosphat | 5 mmol/l |
| | Di-Adenosin-Pentaphosphat | 10 µmol/l |
| | NADP⁺ | 2 mmol/l |
| | N-Acetylcystein | 20 mmol/l |
| | Hexokinase | 3 U/ml |
| | G6P-Dehydrogenase | 3 U/ml |
| | Dithiothreitol | 10 mmol/l |

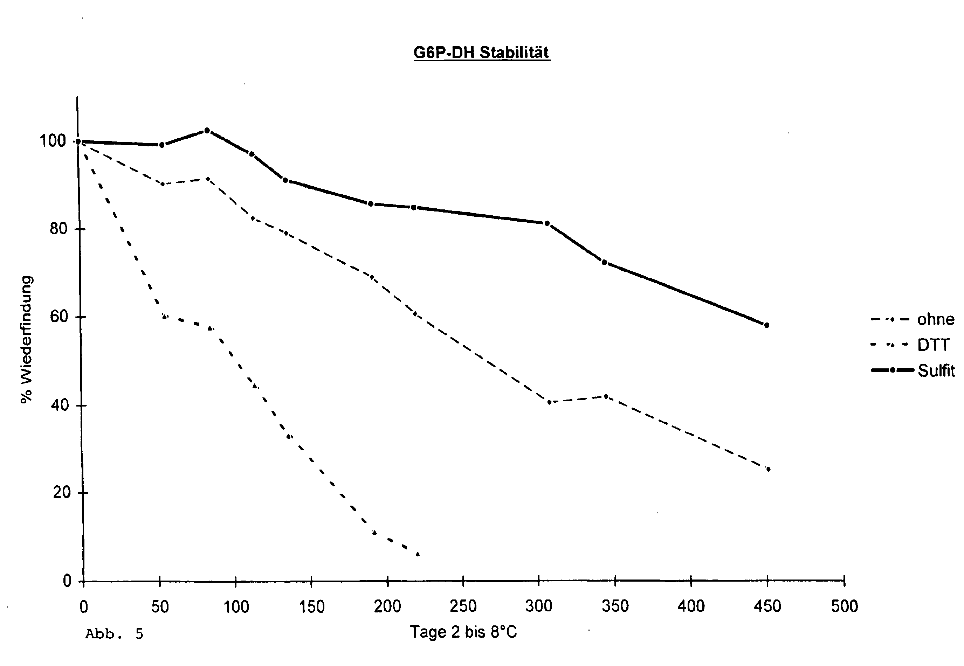

Das Reagenz R wurde wie in Beispiel 1 beschrieben mit der zu vermessenden Probe vereinigt und entsprechende Enzymaktivitäten bestimmt (Tabelle 5, Abbildung 5).

**Tabelle 5**

| Langzeitstabilität G6P-DH (10 mmol/l DTT im Vergleich mit 1 mmol/l Na₂SO₃, Beispiel 1) | | | | | | |
|---|---|---|---|---|---|---|
| | ohne | | 1 mmol/l Sulfit | | 10 mmol/l DTT | |
| Tage | Beispiel 1 | | Beispiel 1 | | Beispiel 3 | |
| 2 bis 8°C | U/ml | % | U/ml | % | U/ml | % |
| 0 | 3.84 | 100.0 | 3.68. | 100.0 | 4.67 | 100.0 |
| 55 | 3.46 | 90.3 | 3.65 | 99.2 | 2.81 | 60.2 |
| 85 | 3.51 | 91.4 | 3.77 | 102.3 | 2.69 | 57.6 |
| 114 | 3.16 | 82.4 | 3.57 | 96.9 | 2.07 | 44.4 |
| 136 | 3.03 | 78.9 | 3.35 | 91.0 | 1.55 | 33.1 |
| 192 | 2.64 | 68.8 | 3.15 | 85.5 | 0.52 | 11.1 |
| 220 | 2.34 | 60.4 | 3.12 | 84.7 | 0.29 | 6.2 |
| 308 | 1.55 | 40.4 | 2.98 | 80.9 | - | - |
| 345 | 1.60 | 41.7 | 2.65 | 72.0 | - | - |
| 451 | 0.96 | 25.0 | 2.12 | 57.6 | - | - |

### Beispiel 4: Regenerierung der CK in Reagenz 1

### Reagenzzusammensetzung:

| | | |
|---|---|---|
| Reagenz 1 (R1): | Imidazol (pH 6,6) | 100 mmol/l |
| | Glucose | 1 mmol/l |
| | Magnesiumacetat | 10 mmol/l |
| | EDTA | 2 mmol/l |
| | ADP | 2 mmol/l |
| | AMP | 5 mmol/l |
| | Di-Adenosin-Pentaphosphat | 10 µmol/l |
| | NADP⁺ | 2 mmol/l |
| | N-Acetylcystein | 20 mmol/l |
| | Hexokinase | 3 U/ml |
| | G6P-Dehydrogenase | 3 U/ml |
| Reagenz 2 (R2): | Capso (pH 9,3) | 20 mmol/l |
| | Glucose | 110 mmol/l |
| | Creatinphosphat | 170 mmol/l |
| | N₂SO₃ | 10 mmol/l |

Mischung von R1, R2 und der jeweiligen Probe und die anschließende Bestimmung erfolgte entsprechend Beispiel 1.

**Tabelle 6**

| Regenerierung der CK in R1 durch Zusatz von Sulfit in R2 | | | | | |
|---|---|---|---|---|---|
| R1 | R2 | | | Wiederfindung | |
| Belastung | Sulfit | Probe 1 | Probe 2 | Probe 1 | Probe 2 |
| 14 Tage | [mmol/l] | [U/l] | [U/l] | [%] | [%] |
| Reagenz frisch (Referenz) | | 236 | 513 | | |
| 2-8°C | 0 | 228 | 494 | 96.6 | 96.2 |
| | 10 | 238 | 509 | 100.8 | 99.5 |
| 35°C | 0 | 179 | 382 | 75.8 | 74.4 |
| | 10 | 227 | 486 | 96.2 | 94.7 |

## Patentansprüche

1. Reagenz zur Bestimmung von Creatin-Kinase in biologischem Probenmaterial bestehend aus zwei Teilreagenzien, wobei im ersten Teilreagenz in wässrigem, gepufferten Medium durch Creatin-Kinase umsetzbare Substrate bzw. entsprechende Coenzyme, ein CK-Aktivator sowie gegebenenfalls weitere für eine oder mehrere Folgereaktionen erforderliche Komponenten enthalten sind, **dadurch gekennzeichnet, daß** mindestens ein Dithionit und/oder ein Sulfitsalz von Alkali-oder Erdalkaliionen im zweiten, jedoch nicht im ersten Teilreagenz enthalten ist, wobei das Dithionit und/oder das Sulfitsalz im molaren Unterschuss zu besagtem CK-Aktivator vorhanden ist.

2. Reagenz nach Anspruch 1, **dadurch gekennzeichnet, daß** im zweiten Teilreagenz maximal 20 mmol/l eines Dithionits und/oder eines Sulfitsalzes von Alkali- oder Erdalkaliionen enthalten sind.

3. Reagenz nach Anspruch 1 oder 2, **dadurch gekennzeichnet, daß** in dem Reagenz Glucose, Crcatinphosphat, G6P-Dehydrogenase, Adenosin-5'-diphosphat, ein GILlcose-6-Phosphat bildendes Enzym, NAD⁺ oder NADP⁺ und ein oder mehrere Adenylat-Kinasc-Inhibitor(en) enthalten sind.

4. Reagenz nach einem der Ansprüche 1 bis 3, wobei im ersten Teilreagenz Glucose, G6P-Dehydrogenase, Adenosin-5'-diphosphat, eine Hexokinase, NAD⁺ oder NADP⁺ und ein CK-Aktivator enthalten sind, und im zweiten Teilreagenz Creatinphosphat und mindestens ein Dithionit und/oder ein Sulfitsalz von Alkali- oder Erdalkaliionen enthalten sind.

5. Reagenz nach einem der Ansprüche 3 oder 4, **dadurch gekennzeichnet, dass** die Konzentration an Glucose maximal 5 mmol/l beträgt.

6. Verfahren zur enzymatischen Bestimmung von Creatin-Kinase in biologischem Probenmaterial unter Verwendung eines Reagenzes gemäß der Ansprüche 1 bis 5.

## Claims

1. Reagent for the determination of creatine kinase in biological sample material consisting of two partial reagents wherein the first partial reagent contains substrates and appropriate coenzymes that can be converted by CK, a CK activator and optionally other components required for one or more subsequent reactions in an aqueous buffered medium, **characterized in that** the second but not the first partial reagent contains a dithionite and/or a sulphite salt of alkaline or alkaline earth ions wherein the dithionite and/or the sulphite salt is present in a submolar amount relative to the said CK activator.

2. Reagent according to claim 1, **characterized in that** the second partial reagent contains a maximum of 20 mmol/l of a dithionite and/or of a sulphite salt of alkaline or alkaline earth ions.

3. Reagent according to claim 1 or 2, **characterized in that** the reagent contains glucose, creatine phosphate, G6P dehydrogenase, adenosine-5'-diphosphate, a glucose-6-phosphate-forming enzyme, NAD⁺ or NADP⁺ and one or more adenylate kinase inhibitor(s).

4. Reagent according to one of the claims 1 to 3, wherein the first partial reagent contains glucose, G6P dehydrogenase, adenosine-5'-diphosphate, a hexokinase, NAD⁺ or NADP⁺ and a CK activator and the second partial reagent contains creatine phosphate and at least one dithionite and/or a sulphite salt of alkaline or alkaline earth ions.

5. Reagent according to one of the claims 3 or 4, **characterized in that** the maximum concentration of glucose is 5 mmol/l.

6. Method for the enzymatic determination of creatine kinase in biological sample material using a reagent according to claims 1 to 5.

## Revendications

1. Réactif de détermination de créatine kinase dans des échantillons biologiques, constitué par deux réactifs partiels, dans le premier réactif partiel, dans un milieu aqueux tamponné, sont contenus des substrats capables de réagir sous l'effet de créatine kinase, ou des coenzymes correspondantes, un activateur de créatine kinase, ainsi qu'éventuellement d'autres composants nécessaires pour une ou plusieurs réactions consécutives, **caractérisé en ce qu'**au moins un dithionite et/ou un sulfite d'ions alcalins ou alcalino-terreux est contenu dans le second réactif partiel, mais non dans le premier, le dithionite et/ou le sulfite étant présents d ans une proportion molaire inférieure à celle dudit activateur de créatine kinase.

2. Réactif selon la revendication 1, **caractérisé en ce que** dans le second réactif partiel sont contenus au maximum 20 mmoles/l d'un dithionite et/ou d'un sulfite d'ions alcalins ou alcalino-terreux.

3. Réactif selon la revendication 1 ou 2, **caractérisé en ce que** dans le réactif sont contenus du glucose, du phosphate de créatine, la G6P-déshydrogénase, l'adénosine-5'-diphosphate, une enzyme formatrice de glucose-6-phosphate, NAD⁺ ou NADP⁺ et un ou plusieurs inhibiteurs d'adénylate kinase.

4. Réactif selon l'une quelconque des revendications 1 à 3, dans lequel dans le premier réactif partiel sont contenus du glucose, la G6P-déshydrogénase, l'adénosine-5'-diphosphate, une hexokinase, NAD⁺ ou NADP⁺ et un activateur de créatine kinase, et dans le second réactif partiel sont contenus du phosphate de créatine et au moins un dithionite et/ou un sulfite d'ions alcalins ou alcalino-terreux.

5. Réactif selon l'une quelconque des revendications 3 ou 4, **caractérisé en ce que** la concentration de glucose est au maximum de 5 mmoles/l.

6. Procédé de détermination enzymatique de créatine kinase dans des échantillons biologiques, avec utilisation d'un réactif selon les revendications 1 à 5.
